# EUROPEAN PATENT APPLICATION

(11) **EP 1 435 236 A1**
(43) Date of publication of application: **07.07.2004**
(21) Application number: 02801495.9
(22) Date of filing: 07.10.2002
(51) Int. Cl.: A61K 31/375, A61P 35/00, A61P 35/02, A61P 43/00

(54) **MALIGNANT CELL DIFFERENTIATION INDUCER COMPOSITIONS AND UTILIZATION OF THE SAME**

(30) Priority: 10.10.2001 JP 2001312788
(71) Applicant: Kochi, Mutsuyuki, Matsudo-shi, Chiba 270-2241 (JP)
(72) Inventor: Kochi, Mutsuyuki, Matsudo-shi, Chiba 270-2241 (JP)
(74) Representative: Helbing, Jörg, Dr. Dipl.-Chem.
(86) International application number: PCT/JP2002/010413
(87) International publication number: WO 2003/032979

(57) **Abstract**

The present invention relates to a differentiation inducer composition for inducing differentiation of malignant cells, characterized by comprising mono-sodium 5,6-O-benzylidene-L-ascorbate (SBA) as an active ingredient, and a pharmaceutically acceptable carrier; and a use thereof. SBA is useful as a novel differentiation inducing agent showing an excellent differentiation inducing action and having extremely low toxicity.

## Description

### TECHNICAL FIELD

The present invention relates to a novel differentiation inducer composition for inducing differentiation of a malignant cell. More specifically, the present invention relates to a differentiation inducer composition for a malignant cell, comprising mono-sodium 5,6-O-benzylidene-L-ascorbate as an active ingredient.

Further, the present invention involves a method of inducing differentiation of cancer or tumor tissue generated in a human body into a normal cell by intravenous, subcutaneous or abdominal cavity administration, or oral administration of mono-sodium 5,6-O-benzylidene-L-ascorbate.

Furthermore, the present invention relates to use of mono-sodium 5,6-O-benzylidene-L-ascorbate for production of various medical compositions administered to induce differentiation of a malignant cell into a normal cell.

### BACKGROUND ART

Cancer cells or tumor cells are generated by malignant alteration of normal cells. A new type differentiation inducing agent for inducing differentiation of a malignant cell is under investigation.

However, differentiation inducing agents have not been developed yet, which can be clinically used alone with safety and efficiency. Therefore, there is a strong need for an excellent new differentiation inducing agent with extremely low toxicity, showing a differentiation inducing action of inducing differentiation of a malignant cell, and which can be clinically utilized with safety.

### SUMMARY OF THE INVENTION

The present inventor made studies to explore a strong, safe and new differentiation inducing agent which can satisfy the above-mentioned need. On the other hand, 5,6-O-benzylidene-L-ascorbic acid and its mono-sodium salt are known compounds having extremely low toxicity. In the studies of the present inventor, it has been found that various cancer cells or tumor cells are induced to differentiate by treatment with mono-sodium 5,6-O-benzylidene-L-ascorbate, as exemplified in the experimental examples described later. Further, it has been found that mono-sodium 5,6-O-benzylidene-L-ascorbate (hereinafter, sometimes abbreviated as "SBA") shows an action of inducing differentiation of a malignant cell at extremely low SBA concentrations.

Further, in a recent study of the present inventor, it has been found that various malignant cells in a human body can be induced to differentiate into normal cells by intravenous administration, abdominal cavity administration or subcutaneous injection of SBA in a small dose of 1 mg to 10 mg/body, for example, 3 mg/body.

Further, it has been found by the present inventor that SBA administered at a dose smaller than 10 mg, preferably a dose of 1 to 10 mg/body induces differentiation of cancer cells or tumor cells into normal cells. The present invention has been accomplished based on the above-mentioned discovery and findings.

Therefore, in the first aspect of the present invention, there is provided a differentiation inducer composition for inducing differentiation of a malignant cell into a normal cell, comprising mono-sodium 5,6-O-benzylidene-L-ascorbate as an active ingredient, and a pharmaceutically acceptable carrier.

### BRIEF DESCRIPTION OF DRAWINGS

Fig. 1 is a graph showing a curve of correlation of a cell viability (%) measured in treating IMR-32 cell in Test Example 2 with SBA of various concentrations, and the concentration of SBA, and a curve of correlation of the count value of pseudo-ganglia observed in surviving cells, and the concentration of SBA.
Fig. 2 is a bar graph showing measured values of the CAT activity of IMR-32 cell not treated with SBA in Test Example 2 and the CAT activity of IMR-32 cell after treatment with 125 or 500 µg/ml of SBA.
Fig. 3 is a bar graph showing the number (× 10⁻⁵) of surviving cells of C6 cell in treatment with SBA of various concentrations in Experimental Example 3.

### BEST MODES FOR CARRYING OUT THE INVENTION

A method of producing 5,6-O-benzylidene-L-ascorbic acid is described in "Steroids", vol. 12, 309 (1968) and the like, and other methods of producing 5,6-O-benzylidene-L-ascorbic acid are described in Japanese Patent Application Publication (JP-B) No. 3-33127 and US Patent No. 5,036,103. The anti-cancer activity of mono-sodium 5,6-O-benzylidene-L-ascorbate (SBA) and a method of producing its mono-sodium salt (SBA) are described in JP-B No. 3-33127 and US Patent No. 5,036,103. Mono-sodium 5,6-O-benzylidene-L-ascorbate (SBA) is a compound of the following formula (I):

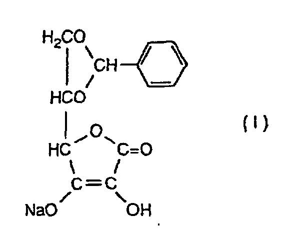

The above-mentioned US Patent No. 5,036,103 describes that when SBA is intravenously administered at a dose of 1.5 to 6 g per day per adult to a patient having a cancer or tumor in pancreas, lymph gland, lung, liver, kidney, stomach, lights or other organs, the total dimension of the carcinoma or tumor in the patient who received this treatment decreases. However, this US patent specification does not describe that SBA has the above-mentioned action of inducing differentiation.

According to the clinical trial made by the present inventor in which patients having a cancer or tumor (35 examples in total, most of them showed cancer advanced stage symptom) were treated by intravenous injection of SBA, the present inventor found that if SBA is intravenously administered at a dose of 1 mg/body to 10 mg/body, preferably at a dose of less than 10 mg/body at a rate of once per day per adult (usually, 40 to 90 kg), SBA can induce differentiation of cancer cells or tumor cells into normal cells efficiently. Further, it has been recognized that also when SBA is administered to a patient showing augmented new blood vessel in eye and eyeground hemorrhage, intravenous administration of SBA at a dose of 1 mg/body to 10 mg/body at a rate of once per day per adult induces differentiation of malignant cells forming new blood vessel into normal cells.

Further, it has also been found that when SBA is administered at a dose of 3 mg/body once per day to a cancer patient with attendant rheumatoid arthritis, the cancer reduces and, at the same time, malignant cells forming new blood vessel in tissue of rheumatoid arthritis lesion are induced to differentiate into normal cells. SBA can be administered, not only intravenously, but also into an abdominal cavity or subcutaneously, or orally.

Thus, the composition of the first aspect of the present invention can be used to administer mono-sodium 5,6-O-benzylidene-L-ascorbate intravenously, subcutaneously or into an abdominal cavity to a cancer bearing patient at a rate of once per day at a dose of 1 mg/body to 10 mg/body effective for inducing differentiation of a cancer cell or tumor cell in cancer or tumor tissue generated in a human body into a normal cell, particularly at a dose of 3 mg/body.

Subject cancers or tumors on which SBA treatment for inducing differentiation of a cancer cell or tumor cell in cancer or tumor tissue into a normal cell is conducted by administering SBA intravenously, subcutaneously or into an abdominal cavity can involve brain tumors including glioblastoma, bladder cancer, maxillary sinus cancer, cervical cancer, goiter, esophageal cancer, stomach cancer, colon cancer, primary hepatic cancer, breast cancer, lung cancer, mediastinal tumor, pancreas cancer, ovarian cancer, uterine cancer, acute leukemia, malignant lymphoma, liposarcoma or femoral head tumor, or prostate tumor.

The differentiation inducer composition according to the first aspect of the present invention is preferably in the dosage form comprising mono-sodium 5,6-O-benzylidene-L-ascorbate, and having a dosage unit per intravenous administration of 1 mg/body to 10 mg/body, preferably 2 mg/body to 4 mg/body, particularly 3 mg/body.

The differentiation inducer composition according to the first aspect of the present invention can be generally prepared in dosage form such as powder, granule, tablet, capsule, syrup, injection, usus externus cream, nasal drop, eye drop and the like by appropriately mixing SBA as an active ingredient with pharmaceutically acceptable additives, for example, pharmaceutically necessary additive components such as carriers, excipients, diluents and the like. The present composition can be administered to a human parenterally or orally.

In preparation of the differentiation inducer composition according to the first aspect of the present invention, SBA as an active ingredient used in the present invention is compounded in an effective amount for attaining a given object of its administration. In general, though the dose of SBA as an active ingredient differs depending on administration route, symptom, and body weight, sex, age and the like of patient, SBA is administered at a dose of about 1 to 10 mg/body, preferably, of 2 to 4 mg/body once per day per adult. However, SBA can be administered continuously or intermittently at a dose in the range not allowing blood SBA concentration to exceed a certain amount by administration of SBA, in view of various conditions such as results of animal experiments and the like. The suitable dosage amount and dosage frequency of SBA under certain conditions should be determined by specialists in view of body weight, symptom and the like of a patient. The content of an active ingredient SBA in preparation of the differentiation inducer composition according to the first aspect of the present invention differs variously depending on dosage form, and usually from 0.1 to 99 wt%, preferably from 0.1 to 90 wt%, more preferably from 0.1 to 50 wt%. For example, in the case of phleboclysis, it is advantageous that injection solution contains SBA usually in an amount of 0.1 to 5 wt%. In the case of oral administration, the composition is used in the form of table, capsule, powder, granule, dry syrup, buccals, solution, syrup and the like together with the above-mentioned solid carrier or liquid carrier. In the case of capsule, tablet, granule and powder, the content of SBA is generally from 3 to 99 wt%, preferably from 5 to 90 wt%, and the remaining portion may be a carrier.

When SBA is administered orally, it is necessary to coat particles of SBA with a water-impermeable coat for protection to give an enteric preparation. This is because when contact with the gastric acid of strong acidity, namely, having low pH, SBA manifests chemical decomposition.

The excipient and carrier to be blended in the composition according to the first aspect of the present invention can be pharmaceutically acceptable compound, and its kind is determined by administration route and administration method. For example, buffer solution having a pH of about 7.4, physiological saline, ethanol, or animal and vegetable oils such as soy bean oil, sesame oil, mineral oil and the like, or synthetic oils and the like are used, as a liquid carrier. Saccharides such as maltose, sucrose and the like, amino acids such as lysine and the like, cellulose derivatives such as hydroxypropylcellulose and the like, polysaccharides such as cyclodextrin and the like, organic acid salts such as magnesium stearate and the like, are used as a solid carrier.

When the differentiation inducer composition according to the present invention is prepared as an intravenous injection, generally desirable as a liquid carrier are physiological saline, buffer solution having a pH of about 7.4, solution of saccharides such as glucose, inositol, mannitol and the like, glycols such as ethylene glycol, polyethylene glycol and the like. Further, it is also possible that SBA is dissolved in physiological saline together with excipients such as saccharides such as inositol, mannitol, glucose, mannitol, maltose, sucrose and the like and amino acids such as phenylalanine and the like, and the resulted solution is subsequently made into a lyophilized preparation. This can be used by dissolving, in administration, in a solvent suitable for injection, for example, a diluent for intravenous administration such as sterile water, physiological saline, buffer solution of pH 7.4, glucose liquid, electrolyte solution, amino acid aqueous solution and the like.

Furthermore, according to the second invention in another aspect of the present invention, the present invention involves use of mono-sodium 5,6-O-benzylidene-L-ascorbate for production of a differentiation inducer composition administered to induce differentiation of a malignant cell which is a cancer cell or tumor cell in cancer or tumor tissue generated in a human body. Here, cancers or tumors which are subjected to which the composition is administered can involve, for example, maxillary sinus cancer, cervical cancer, goiter, esophageal cancer, stomach cancer, colon cancer, primary hepatic cancer, breast cancer, lung cancer, mediastinal tumor, pancreas cancer, ovarian cancer, uterine cancer, acute leukemia, malignant lymphoma, liposarcoma, femoral head tumor, bladder cancer or brain tumor, particularly, glioblastoma, or prostate tumor.

Furthermore, in the third aspect, the present invention involves a method of inducing differentiation of cancer or tumor tissue into a normal cell, consisting administering mono-sodium 5,6-O-benzylidene-L-ascorbate intravenously, subcutaneously or into an abdominal cavity at a dose of 1 mg/body to 10 mg/body, preferably of 2 mg/body to 4 mg/body, particularly a dose of 3 mg/body at a rate of once per day to a patient having a cancer cell or tumor cell of cancer or tumor tissue generated in a human body.

In the above-mentioned medical method according to the third aspect of the present invention, it is found that also when SBA is injected intravenously or subcutaneously at a dose of 1 mg/body to 10 mg/body, preferably a dose of 3 mg/body per dosage, a differentiation inducing effect of inducing differentiation of a malignant cell of a cancer or tumor is obtained.

### EXAMPLES

Hereinafter, the differentiation inducer composition according to the first aspect of the present invention and the differentiation inducing method for a malignant cell using SBA as a differentiation inducing agent according to the present invention will be specifically illustrated with reference to the following examples and test examples.

First, preparation examples of the differentiation inducer composition according to the first aspect of the present invention are shown in the following Examples 1 to 3. However, the composition according to the first aspect of the present invention is not limited to the following Examples 1 to 3.

### Example 1: Preparation of tablet

The following ingredients were mixed uniformly.

| | |
|---|---|
| SBA (namely, mono-sodium 5,6-O-benzylidene-L-ascorbate) | 50 g |
| Magnesium metasilicate aluminate | 14 g |
| Corn starch | 21 g |
| Lactose | 35 g |
| Crystalline cellulose | 60 g |
| Carboxymethylcellulose (CMC) calcium salt | 18 g |

To the resulted mixture was added 2 g of magnesium stearate and mixing thereof was further continued. The resulted powdery mixture was tabletted, to obtain tablets of a weight of 200 mg per tablet. The tablets were surface-coated with polyvinylacetal diethylaminoacetate as a film coating agent which is insoluble in stomach but soluble in intestinal canal. By this, an enteric coated tablet was prepared. If necessary, it may be colored with an edible coloring agent.

### Example 2: Preparation of capsule

The following ingredients were uniformly mixed.

| | |
|---|---|
| SBA | 1000 g |
| Lactose | 960 g |
| Magnesium stearate | 40 g |

The resulted powdery mixture was filled in hard gelatin capsules, so that each capsule included the powder each in an amount of 200 mg.

### Example 3: Preparation of injection

The following components were prepared.

| | |
|---|---|
| SBA | 1 mg |
| Sterile water | 50 mg |

One (1) g of the above-mentioned SBA was dissolved in 50 ml of sterile water. The resulted solution was filtrated through a micro-pore membrane for bacterial elimination. The filtrated liquid was divided into 10 ml volume brown vials each at a proportion of 5 mg/0.25 ml under an aseptic condition, and aseptically freeze-dried, then, the vial was filled with a nitrogen gas.

Thus, a SBA lyophilized preparation for intravenous injection was obtained. The preparation in the vial could be completely dissolved immediately when 5 ml of sterile physiological saline was added under aseptic condition directly before use for intravenous injection. The resulted aqueous solution contained SBA in a concentration of 1 mg/ml, and was suitable for intravenous administration.

### Experimental Example 1: Evaluation of acute toxicity

An aqueous solution prepared by dissolving SBA in sterile distilled water was intravenously injected at an amount of 4 ml/kg from the caudal vein of Wistar rats. SBA was intravenously injected at a dose of SBA administered to the rat of 500 mg/kg, 1000 mg/kg, 1500 mg/kg and 2000 mg/kg. The mortalities of the treatment group of the rats received SBA in different doses were evaluated. By this, it was recognized that the LD₅₀ value of SBA when intravenously administered to the Wister rat was 1693 mg/kg (confidence interval: 1306 to 2194 mg/kg).

In the same manner as in the above-mentioned toxicity test, an aqueous solution of SBA was intravenously administered from the caudal vein of NMR1 mice. It was recognized that the LD₅₀ value of SBA when intravenously administered to the NMR1 mouse was 1815 mg/kg (confidence interval: 1399 to 2858 mg/kg).

### Experimental Example 2

This example is a test in which human neuroblastoma IMR-32 cells which are one example of neuroblastomas belonging to sympathetic tumors are, when treated with SBA, induced to differentiate into normal neurocytes forming a ganglion-like cell coagulate (Pseudoganglia) having a nature of a cholinergic neurocyte, and exemplifies that SBA has a differentiation inducing action.

### (a) Material used in testing

SBA is used as a solution dissolved in buffer saline [hereinafter, abbreviated as PBS(-)] comprising no Ca²⁺/Mg²⁺. As [³H] acetyl-coenzyme A (acetyl-CoA), a product manufactured by Amersham was used, and as propidium iodide, acridine orange and choline chloride, products manufactured by Sigma were used. As fetal bovine serum (hereinafter, abbreviated as FCS), a product manufactured by Cansera International was used. The neuroblastoma IMR-32 cell to be tested was obtained from Japanese Cancer Research Resource Bank (JCRB), cultured in PRMI 1640 medium (manufactured by Nissui Pharmaceutical Co., Ltd.) supplemented with 10% of FCS, and maintained in air containing 5% of CO₂ at 37°C.

### (b) Measurement of survival rate of cell after treatment with SBA

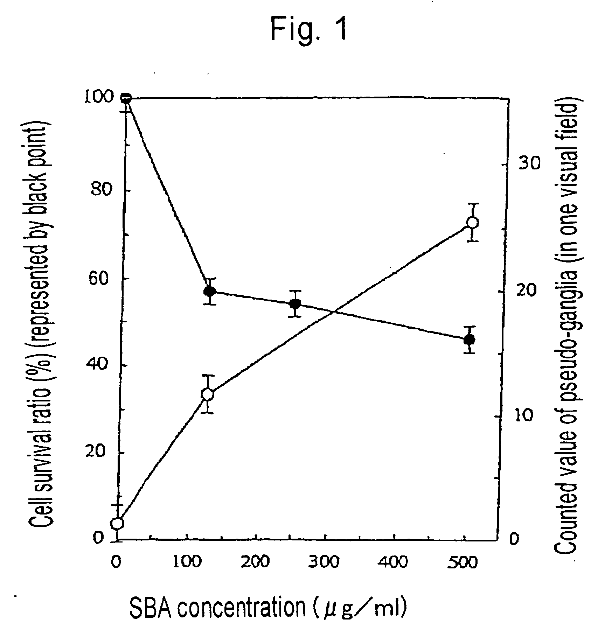

IMT-32 cells were inoculated on a medium in a schale in an amount of 1 × 10⁵ cells/schale, cultured for 24 hours, then, treated with SBA of various concentrations for 48 hours. The survival rate of the cells was measured by 0.2% of trypan blue exclusion test. The measured value (represented by black point) of the survival rate (%) by the 0.2% of trypan blue exclusion test method is shown in Fig. 1 herein appended, and represents average value ± standard error (SE) of three continuous tests. The above-mentioned test was conducted in the absence of SBA, as a control test.

### (c) Evaluation of formation of pseudo-ganglion of cell after treatment with SBA

IMR-32 cells cultured for 48 hours in the presence of 125 g/ml of SBA or 500 g/ml of SBA. Morphological change of the cells, and the number of pseudo-ganglia formed were observed under a phase-contrast microscope (magnification: 200).

It was recognized that morphological change occurs so that IMR-32 cells form pseudo-ganglia by treatment with SBA. The counted value of pseudo-ganglia in one visual field of a phase-contrast microscope is represented by a white point on a curve in Fig. 1.

In the above-mentioned observation, it was recognized that human neuroblastoma IMR-32 cells were induced to differentiate into normal neurocytes by the action of SBA.

### (d) Assay of choline acetyl transferase (hereinafter, abbreviated as CAT) of cell after treatment with SBA

IMR-32 cells were treated for 6 days using SBA of various concentrations. Then, the cells after treatment with SBA were harvested, washed with PBS(-) twice, and the cells were dissolved in 1 M of NaCl-2.5% of Triton X-100. The CAT activity of the resulted cell lysate was analyzed by a modified method of the method by Fonnum, Rabinosky et al. In this analysis, a reaction mixture (100 1) was prepared so that it contained 50 mM of potassium phosphate (pH 6.8), 0.2 M of NaCl, 8 mM of choline chloride, 1 mM of EDTA, 0.1 mM of physostigmine sulfate, 0.5% of Triton X-100, 25 µM of [³H] acetyl-CoA (40 mCi/mMol) and 100 µg of the cell lysate. The above-mentioned reaction mixture was culture at 37°C for 1 hour. The reaction was terminated by adding 300 µl of 50 mM of potassium phosphate-1 mM of EDTA, then, immediately adding 1 ml of acetonitrile containing 5 mg of tetraphenylboronsodium and 2 ml of toluene. The resulted mixture was shaken for 3 minutes, then, centrifugally separated at 3000 × g for 10 minutes. The resulted organic solvent layer (usually, 1.5 ml) was mixed with 10 ml of a toluene-scintillator mixture, and its radioactivity was measured by a liquid-scintillator sectrometer (manufactured by Beckman, LS5000TD).

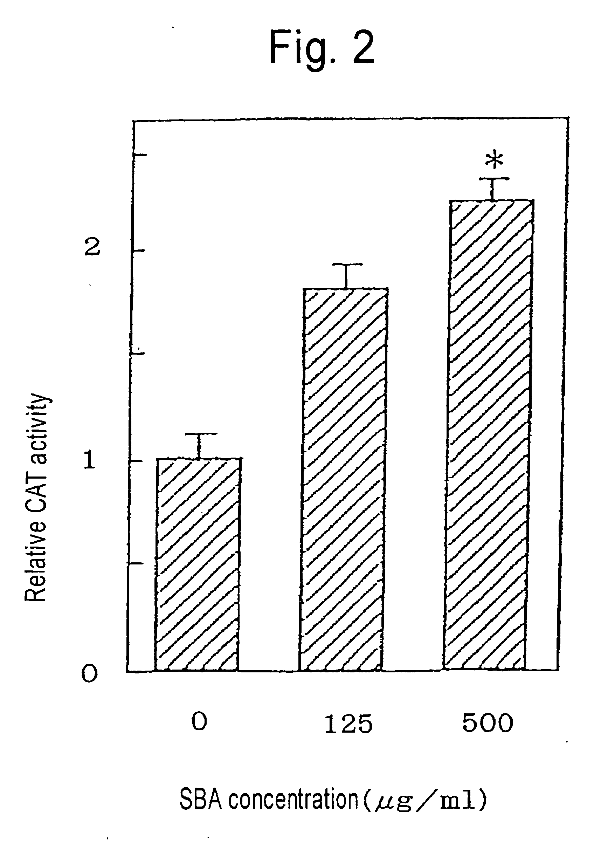

The measured value of the CAT activity assayed from the amount of thus incorporated radioactive acetyl-CoA is shown as a bar graph in Fig. 2. This measured value represents average value ± standard error (SE) of three continuous tests [*P<0.002 (comparison with contrast)].

It is recognized that surviving cells after treatment with SBA show CAT activity significantly higher as compared with un-treated IMR-32 cells, having a nature of cholinergic neurocytes.

From the results of the above-mentioned tests, the effect of SBA on human neuroblastoma IMR-32 cells is understood as described below.

Namely, the survival ratio (%) of surviving cells after treatment with SBA was suppressed in a dosage-dependent manner by continuous treatment for 8 hours by SBA, and its CD₅₀ was calculated to be 462 g/ml (see, Fig. 1). The cell survival ratio decreased depending on the treatment time, and on day 6 after treatment with SBA of various concentrations, a decrease of about 6% was still observed as compared with the survival ratio on treatment for 48 hours.

In surviving cells, morphological change was sufficiently observed, and it is recognized that this morphological change represents formation of large cell coagulates (pseudo-ganglia) and some of the cell coagulates mutually communicate by long spreading neuritis. Emersion of pseudo-ganglia varies depending on the dose of SBA (see, Fig. 1). In the case of treatment with 500 µg/ml of SBA, it was observed that pseudo-ganglia mutually collided and a lot of neuritis extended. Though correct measurement of the ratio of cells surviving in the cells treated with SBA and differentiated was difficult due to formation of cell coagulates, nonetheless, at least 20 to 30% of survived cells were cells in the case of treatment with 500 µg/ml of SBA, and believed to be induced to differentiate into neurocytes having neuritis. To determine whether the cells differentiated by treatment with SBA were cholinergic neurocytes or not, the CAT activity of surviving cells after treatment with SBA for 6 days in the above-mentioned (d). The CAT activity of surviving cells treated with 500 µg/ml of SBA is higher 2.2-fold than the CAT activity of control (un-treated) cells, the extent of its increase is dosage-dependent (see, Fig. 2), showing that the cells differentiated by treatment with SBA acquired a nature of cholinergic neurocytes.

By separate tests, it was recognized that cells died in treatment with SBA were by apoptosis and necrosis.

### Experimental Example 3

This example exemplifies a test in which rat glioma C6 cells are induced to differentiate into astro cytes by treatment with SBA.

A rat glioma C6 cell which is one example of the central nerve system tumors is known to have both glial feature and neuronal feature, and it is known that the C6 cell, when treated with GAMP or sodium butyrate, differentiates into highly mature astrocyte having high level of expression of glial-livelialy acidic protein (hereinafter, GFAP) which is a marker for astrocytes.

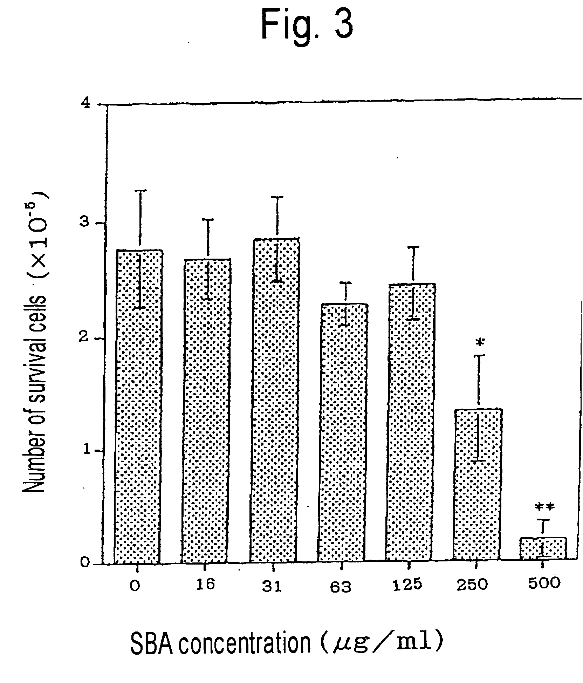

Rat glioma C6 cells obtained from Japanese Cancer Research Resource Bank (JCRB) were inoculated on a medium in an amount of 1 × 10⁵ cells/schale, cultured for 24 hours. Then, the resulted cultured cells were treated with SBA of various concentrations for 3 days. Then, the cells were dyed with 0.2% trypan blue, then, the number of surviving cells was counted. The counted value of cell number represents average value ± standard error (SE) of three continuous tests [*P<0.00301 and *P<0.00001 (in comparison with contrast)]. The counted value is shown as a bar graph in Fig. 3. This test was conducted in the absence of SBA, as a control test. As apparent from Fig. 3, proliferation of C6 cells was suppressed in a dosage-dependent manner by SBA, and CD₅₀ of SBA was calculated to be 250 µg/ml.

Further, C6 cells were cultured for 3 days in the presence of 125 µg/ml of SBA, and morphological change of the cells was observed under a phase-contrast microscope. Formation of astrocytes was recognized, and these cells had a form of elongated cells.

The C6 cells were treated continuously with 250 µg/ml SBA for 3 days or 6 days. Then, the level of expression of GFAP of cells after treatment with SBA was judged by a Western blot method using a monoclonal antibody against GFAP (clone G-A-5; manufactured by Boehringer Mannheim). The level of expression of GFAP in cells after treatment with SBA for 6 days was recognized to be higher 6.7-fold than the GFAP expression level of the control cell, showing that the C6 cells differentiated into mature astrocytes.

Also in this test example, it was clarified that SBA has an action capable of inducing differentiation of astrocytes into normal neurocytes.

### INDUSTRIAL APPLICABILITY

As described above, it was found that mono-sodium 5,6-O-benzylidene-L-ascorbate (SBA) which is a known low-toxic compound has a differentiation inducing action capable of converting malignant cells such as cancer cells or tumor cells and malignant cells forming angiogenesis respectively into normal cells, in the present invention. The above-mentioned SBA is useful as a novel differentiation inducing agent having an excellent differentiation inducing action and having extremely low toxicity.

## Claims

1. A differentiation inducer composition for inducing differentiation of a malignant cell, comprising mono-sodium 5,6-O-benzylidene-L-ascorbate as an active ingredient, and a pharmaceutically acceptable carrier.

2. The composition according to Claim 1, comprising mono-sodium 5,6-O-benzylidene-L-ascorbate to be administered at a dose unit of 1 to 10 mg/body per dosage per day for intravenous administration of the composition.

3. The composition according to Claim 1, wherein mono-sodium 5,6-O-benzylidene-L-ascorbate is administered intravenously, subcutaneously or into an abdominal cavity to a cancer patient at a rate of once per day at a dose of 1 to 10 mg/body effective for inducing differentiation of a cancer cell or tumor cell in cancer or tumor tissue generated in a human body, and the composition comprises mono-sodium 5,6-O-benzylidene-L-ascorbate as an active ingredient.

4. The composition according to Claim 3, wherein the cancer or tumor described in Claim 3 is maxillary sinus cancer, cervical cancer, goiter, esophageal cancer, stomach cancer, colon cancer, primary hepatic cancer, breast cancer, lung cancer, mediastinal tumor, pancreas cancer, ovarian cancer, uterine cancer, acute leukemia, malignant lymphoma, liposarcoma, femoral head tumor, bladder cancer or brain tumor, particularly, glioblastoma, or prostate tumor.

5. Use of mono-sodium 5,6-O-benzylidene-L-ascorbate for production of a differentiation inducer composition administered to induce differentiation of a cancer cell or tumor cell in cancer or tumor tissue generated in a human body.

6. The use according to Claim 5, wherein the cancer or tumor described in Claim 5 is maxillary sinus cancer, cervical cancer, goiter, esophageal cancer, stomach cancer, colon cancer, primary hepatic cancer, breast cancer, lung cancer, mediastinal tumor, pancreas cancer, ovarian cancer, uterine cancer, acute leukemia, malignant lymphoma, liposarcoma, femoral head tumor, bladder cancer or brain tumor, particularly, glioblastoma, or prostate tumor.

7. A method of inducing differentiation of cancer or tumor tissue generated in a human body, comprising administering mono-sodium 5,6-O-benzylidene-L-ascorbate intravenously, subcutaneously or into an abdominal cavity to a patient having cancer or tumor tissue generated in a human body once per day at a dose of 1 mg/body to 10 mg/body.

8. The method according to Claim 7, wherein the cancer or tumor described in Claim 7 is brain tumor, particularly, glioblastoma, bladder cancer, maxillary sinus cancer, cervical cancer, goiter, esophageal cancer, stomach cancer, colon cancer, primary hepatic cancer, breast cancer, lung cancer, mediastinal tumor, pancreas cancer, ovarian cancer, uterine cancer, acute leukemia, malignant lymphoma, liposarcoma or femoral head tumor, or prostate tumor.
